# EUROPEAN PATENT SPECIFICATION

(11) **EP 2 873 329 B1**
(45) Date of publication and mention of the grant of the patent: **17.05.2017**
(21) Application number: 14003635.1
(22) Date of filing: 27.10.2014
(51) Int. Cl.: A23C 9/146, A23J 1/20, C07K 14/79, C07K 1/18, C12N 9/08

(54) **Process of whey protein separation from milk medium**
Verfahren zur Molkeproteintrennung aus einem Milchmedium
Procédé de séparation de protéines de lactosérum à partir d'un support de lait

(30) Priority: 15.11.2013 CZ 20130885
(43) Date of publication of application: 20.05.2015
(73) Proprietor: Univerzita Palackého, 771 47 Olomouc (CZ)
(72) Inventor: Holá, Katerina, 779 00 Olomouc (CZ); Zboril, Radek, 779 00 Olomouc (CZ); Medrik, Ivo, 783 44 Drahanovice (CZ)
(74) Representative: Hartvichova, Katerina

(56) References cited:
- EP-A1- 2 272 378
- EP-A2- 0 352 678
- EP-A2- 1 087 828
- CN-A- 101 032 671
- US-A- 4 791 193
- US-A- 4 976 865
- US-A- 5 516 675
- GEOFFREY N. BROWN ET AL: "Multi-cycle recovery of lactoferrin and lactoperoxidase from crude whey using fimbriated high-capacity magnetic cation exchangers and a novel "rotor-stator" high-gradient magnetic separator", BIOTECHNOLOGY AND BIOENGINEERING, vol. 110, no. 6, 22 June 2013 (2013-06-22) , pages 1714-1725, XP055174347, ISSN: 0006-3592, DOI: 10.1002/bit.24842

## Description

### Field of invention

The invention relating to the field of dairy industry based on the process for separation of whey proteins, e.g. lactoferrin and lactoperoxidase, from milk medium, especially from fresh milk and its technological solution.

### Background Art

Lactoferrin (Lf) and lactoperoxidase (LPO) are whey proteins having the same molecular weight of 78 kDa. Both proteins are mostly presented in colustrum where they act as immunity protectors of newborns from attack of various pathogens.

Lf is known as antibacterial, antiviral and antimicrobial protein [Levay. P.F.; Viljoen, M. Hematol. J. 1995, 80, 252-267]. At the same time Lf proves antioxidant and antitumor effects [(a) Gutteridge, J. M.; Paterson, S. K.; Segal, A. W.; Halliwell, B. Biochem. J. 1981, 199, 259-261, (b) Tuccari, G.; Barresi, G. Biometals 2011, 24, 775-784]]. Generally, the bioactive effects of Lf support the human immunity. There exist a lot of pharmaceutical products and food supplements on the market containing Lf. Usually, Lf is added into the products for boosting the immunity, it is also a component of products against acne, urinary infections or anemia, and it is also a part of products against inflammatory diseases of gastrointestinal tract. Lf is also commonly sold as food supplement and is an important component of nurse nutrition.

LPO acts in the human body as an antibacterial or as an antioxidant agent [Ostdal, H.; Bjerrum, M. J.; Pedersen, J. a; Andersen, H. J. J. Agric. Food Chem. 2000, 48, 3939-3944]. LPO is typically added into the cosmetics, tooth pastes or mouthwashes to treat periodontal diseases.

Thanks to the broad applications of both proteins, wide variety of techniques for their large scale separation has been developed. The increasing demands on quality and purity have led to development of more sophisticated and effective separation processes. Generally Lf and LPO are mostly isolated from bovine milk. The concentration of Lf in bovine milk is aprrox.100 mg.l⁻¹, while concentration of LPO is about 30 mg.l⁻¹. A considerable advantage of Lf is its high thermostability during the separation process [Sanchez, L.; Peiro, J. M.; Castillo, H.; Perez, M. D.; Ena, J. M.; Calvo, M. J. Food Sci. 1992, 57, 873-879]. Thanks to it, Lf can be isolated also from whey, waste product of dairy industry. However, the concentration of Lf in whey is considerably lower (almost eight times) than in fresh milk. On the other hand, less stable LPO is usually present in whey in denatured, i.e. inactive form.

The US patent 4 436 658 is focused on the separation of Lf and immunoglobulins (Ig) from whey using column adsorption on silica-gel in basic conditions (pH 7.9-8.5). Lf is afterwards eluted from silica-gel by changing the ionic strength (I) in to the low pH values; e.g. I = 0.5 M, pH 4. By using this method, Lf is eluted together with Ig, therefore the obtained Lf shows the purity about 70 %, but usually only 50 %. Such low purity and recovery yield per liter of milk is inconvenient for practical usage.

The separation of Lf (LPO) is often provided by ion exchange chromatography. The principle of this purification method is based on the fact that in milk/whey at neutral pH only these two minor proteins (Lf and LPO) are positively charged (they pI values are higher than 7). Therefore any other purification methods, for example a separation on silica-gel, gel chromatography, affinitive chromatography or isolations based on specific interaction with antibodies are minimally used.

Separation of Lf using ion-exchange chromatography is described in the patent US 4 791 193. Herein, the detailed analysis of various types of ion-exchange adsorbents from the viewpoint of capacity together with the purity of isolated Lf is provided. As the most suitable adsorbents have been identified ion-exchangers composed from (bio)polymer materials, such as agarose, agarosadextran, hydoroxylated polymethacrylate etc. All of them reveal weakly acidic carboxylate function on their surface. One of the major advantages of these adsorbents is their high porosity, guaranteeing the high capacity, and hydrophilic character, preventing denaturation of separated proteins. Bounded Lf and LPO are eluted by changing the ionic strength. Lf is isolated from whey but also from non-pasteurized skim milk. However in dairy practice it is almost impossible to change the technical process in the sense that pasteurization would be provided after skimming phase. Besides that, this patent does not take into consideration LPO bounded to the sorbent together with Lf during the isolation and a possible contamination of Lf by LPO.

Separation of Lf and LPO by ion-exchange chromatography is described in detail in the patent US 5 516 675. Herein, the conditions in which individual proteins bounded on the sorbent can be eluted are discussed. The authors used various pH values and different values of ionic strength. At first, LPO is eluted at pH lower than 5 by using ionic strength up to value 0.5 M. Then at pH around 7 and *I* ≤ 0.5 M Ig are eluted, however only small amount of Ig are bounded, since most of them have their isoelectric point higher than 7 and generally their concentration in milk is negligible. Afterward, Lf is isolated at pH around 7 and *I* = 1 M. However, the purity of isolated fractions (80% and higher), established by SDS-PAGE (polyacrylamide gel electrophoresis in the presence of sodium dodecylsulfate) is questionable. From the SDS-PAGE it is enable to distinguish between Lf and LPO. Moreover the potential usage of buffers during the elution could be problematic in large-scale conditions.

Another relevant patent, US 5 596 082, describes the above mentioned methods while the Lf/LPO separation is performed on ion-exchange sorbent with size bigger than 100 µm, typically 300 µm (elution is carried out by gradient ionic strength, neutral pH). Thanks to appropriate parameters of column sorbent the whey could pass through the column with high speed (600 times of column volume per hour). This high flow rate is beneficial in separations where the separated agent is presented in media in small concentrations as Lf and LPO in milk. However, this solution does not offer the separation from unpasteurized milk media.

Another way how to perform fast isolation of Lf and LPO is membrane separation. During this separation process, the whey pass through the systems of membranes which are functionalized with typical ion-exchange functional groups (-OS0₃H, -COOH etc.). This separation method is described in the publication Isolation of proteins (Hatti-Kaul, R. & Mattiasson, B. Marcel Dekker, Inc.: New York, 2003). It is more difficult from the viewpoint of preprocessing of whey or milk medium because the blocking of separation unit could occur more often than in column arrangement. Thus, separation from fresh milk is almost impossible.

Further patents focusing on large-scale separation of Lf and LPO only extend the processes and methods described above. For example in the US patent 7 932 069 the production of purer LPO was reached due to the precipitation of impurities during the ultrafiltration. Another example is the US patent 5 919 913 where the separation of human Lf from milk is solved by using transgene modified cows. Important part of production of these proteins is purification of obtained solutions by ultrafiltration followed by drying or lyofilization (to form powder). These processes are good to combine in order to prevent degradation of quality, i.e. denaturation of purified proteins. As an example of such purification process it can be mentioned the solution of production line, according to the work CZ 23725 U1 where optimization of mentioned processes leads to minimal sample degradation.

It seems that well-established separation processes provide the fraction of Lf and LPO of sufficient quality. However, the current key parameter is the recovery ratio of these proteins to volume of treated milk, as well the quality of Lf and LPO. Both of these parameters depend mainly on the type of medium used for isolation. The advantage of isolation from whey is the low price, on the other hand the Lf concentration is low and LPO occurrence is negligible. Although the Lf concentration in skim milk is high, the quality and concentration of LPO is depending on the type of pasteurization. In dairy industry is more convenient to perform the pasteurization which fully denaturizes LPO. Moreover, the skim milk must undergo a complicated preprocessing before separation, in order to avoid blocking of column or membranes.

The separation medium is an issue in the work US 2005/0220953 A1. This patent describes how to get concentrated Lf solution during the milk treatment. Low Lf concentration in whey is the consequence of casein precipitation during the production of cheese and cottage cheese. Since the Lf has opposite charge to casein they strongly interact together by ionic strength and bound Lf sediments with the precipitated casein. This patent application describes how to release the bound Lf from casein by ionic strength e.g. by using NaCI solutions. The obtained solution must undergo preprocessing before the chromatography in order to get out the rest of NaCI. The precipitated casein needs to be also purified from the NaCI solution before its further utilization. Thanks to above mentioned process the recovery ratio is increased and quite a good concentrated solution of Lf can be obtained.This leads to shorted time of chromatographic separation, but on the other hand the process is technologically very demanding. Moreover, in such process only limited amount of already pasteurized milk can be used.

In order to fulfill all requirements for effective Lf and LPO separation (purity, quality and sufficient recovery), a suitable way for isolation seems the usage of ion-exchange chromatography directly from fresh milk. However the fresh milk contains the fat-globules (up to 10 µm in size) blocking the separation unit (column, membrane). There exist some papers in literature where the fresh milk is applied on the column at 37 °C [C.J. Fee and A. Chand, Sep. Purif. Technol., 2006, 48, 143-149]. At this temperature the fat passes into liquid and does not block the column. However, in industry the heating of milk on 37°C is unreal due to possible bacteria proliferation.

Separation of LPO and Lf is also very often conducted by expanded bed absorption. This technique is based on sorbent with high density that quickly sediment in gravitation field. The separation media is pumped upwards the sedimentation of the particles and the particles are kept in fluidized bed for fast absorption from feedstock media. EP 2272378 discloses sorbents with non-magnetic stainless steel core for expanded bed adsorption. WO 99/65586 and EP 1087828 also relate to expanded bed adsorption of proteins in general. US 5516675 discloses separation of Lf with purity of 92-95 %, as measured by SDS-PAGE method, which is not able to distinguish between Lf and LPO. Brown et al.: Biotechnology and Bioengineering, vol. 110, 2013, 1714-1725 uses magnetic non-porous sorbent, thereby achieving low yields and purity of Lf. CN101032671, US 4976865 and EP 0352678 disclose various types of apparatuses suitable for whey protein separation by adsorption on sorbents.

The reasonable way how to separate Lf and LPO from fresh medium is the separation from milk protein concentrate (MPC). MPC is prepared by combination of microfiltration and ultrafiltration. The microfiltration separates the fat and casein which are further processed. The passed whey proteins are concentrated by ultrafiltration in order to obtain suitable volume of medium for Lf and LPO separation. However, this technological procedure is extremely costly and process demanding and it is complicated to implement it into dairy practice.

Another potential alternative for isolation of proteins is magnetic separation with a possibility to separate by batch procedure; the advantage is that the substances are isolated just by mechanical stirring of the sorbent in whole volume of the medium. Thanks to this possibility the separation can be performed in heterogeneous media and the magnetic sorbent can be afterwards take out just by external magnetic field. The medium does not have to pass through the whole separation unit (column, membrane); this technological step is typically time-dependent on the volume of the medium. Therefore, the magnetic separation seems as a time-effective approach that can be used even in non-homogenous medium.

The material used for separation of various bioactive substances is typically nanoparticles or microparticles based on iron oxides (Fe₃O₄, γ-Fe₂O₃); these particles are usually commercially available or can be prepared by simple synthetic approaches. There exists a large spectrum of their surface functionalization. There can be attached various types of polymer substances or substances with surface activity that can be modified in the following steps. The well-known separation processes for magnetic separation and purification of proteins are reviewed in the literature [Safarik, I.; Safarikova, M. BioMagn. Res. Technol. 2004, 17, 1-17].

The fundamental work in development of magnetic separation techniques was made in Karlsruhe in Germany and it is very well described in US patent 6942806. This method is called High Gradient Magnetic Fishing (HGMF). It is a specific method for separation of magnetic particles based on flowing of the particles through switchable permanent magnet. An important issue in HGMF is the magnetic sorbent. It has to accomplish the following parameters: the particles should exhibit low porosity for fast adsorption of targeted substances (up to 5 minutes), superparamagnetism (no remanent magnetization) connected with good dispersibility after separation. Further parameters are high surface area for higher capacity (20 až 100 m2·g⁻¹) and the size of these particles (aggregates) should be bigger than 500 nm for faster response on magnetic field or for possibility of mechanical separation by filters

[Franzreb, M.; Siemann-Herzberg, M.; Hobley, T. J.; Thomas, O. R. T. Appl. Microbiol. Biotechnol. 2006, 70, 505-516].

This technique has been also used for separation of Lf and LPO. Nanoparticles of iron oxide in the form of superparamagnetic aggregates prepared by precipitation technique by Massart were used as magnetic sorbent. These aggregates were functionalized by strongly acidic sulfonate group (-OSO₃H) by demanding synthetic procedures. The best capacity for model protein (lysozyme) was 230 mg of protein per 1 gram of particles at room temperature. However, the purity of prepared Lf was insufficient because it was not possible to isolate Lf and LPO separately [(a) Heebøll-Nielsen, A.; Justesen, S. F. L.; Hobley, T. J.; Thomas, O. R. T. Sep. Sci. Technol. 2004, 39, 2891-2914, (b) Heebøll-Nielsen, A.; Justesen, S. F. L.; Thomas, O. R. T. J. Biotechnol. 2004, 113, 247-262].

Another technique which was used for separation of Lf is affinity magnetic separation via magnetic nanoparticles with attached heparin. The particles had the capacity of 164 mg of Lf per 1 gram of particles at 37 °C. Hence, thanks to the specific interaction they were able to adsorb just Lf which was afterwards eluted just by solution of sodium chloride. [Chen, L.; Guo, C.; Guan, Y.; Liu, H. Sep. Purif. Technol. 2007, 56, 168-174]. However, the low capacity and high-cost preparation of these particles do not allow an expansion of this process into the large-scale.

There can be concluded that magnetic separation based on batch separation has a big potential for large-scale isolation of biomacromolecules from crude and inhomogeneous media. This separation technique has a potential to overcome the traditional chromatographic techniques thanks to its process simplicity and low requirements for pretreatment of the separation medium. However, up until now magnetic separations of Lf did not allow to prepare Lf of high purity with low economic and technological requirements, and at the same time the process would allow to separate Lf from fresh medium in large-scale. The aim of this invention is to show a new strategy of Lf and LPO separation which allow isolation from various types of milk media. Subsequently, the method can be easily implemented in to the dairy factories producing various types of dairy products.

### Summary of the invention

The aim of the presented invention is a process for separation of whey proteins lactoferrin and lactoperoxidase, from milk media, preferably from fresh milk or whey, by sorbents, wherein the milk medium is in contact with the sorbent by mechanical stirring; the sorbent is porous and magnetically filterable, made from polymer material functionalized by cation-exchange groups selected from -COO⁻, -OSO₃⁻, -PO₃²⁻, and -OPO₃²⁻, and the sorbent contains a magnetic part composed of inorganic metal compounds or zero-valent metals, said sorbent is kept in suspension in a separate filtration space with filtration mechanism, preferably in filtration basket, and adsorbs whey proteins, whereupon the filtration mechanism with adsorbed proteins is taken out and after wash-out of unattached proteins and other unwanted parts of milk medium the attached whey proteins are eluted, wherein after the removal of unattached proteins by deionized water the adsorbed whey proteins are released from the sorbent by ionic strength,

in the case that the milk medium contains lactoperoxidase and lactoferrin, lactoperoxidase and impurities such as immunoglobulins are eluted in the first step by ionic strength 0.01-0.3 mol.dm⁻³, then the residual lactoperoxidase is eluted by ionic strength 0.05-0.5 mol.dm⁻³ and afterwards lactoferrin by ionic strength 0.5-5 mol.dm⁻³;
and afterwards processed into the powder form.

The process of adsorption of whey proteins is performed for the period of time 0.1- 24 hours, 2-6 hours in optimum, at the temperature 1-85 °C, using the sorbent with cation-exchange groups as -COO⁻ or -OSO3⁻ or -PO₃²⁻ or -OPO₃²⁻, and the sorbent contains a magnetic part composed by inorganic metal compounds or zero-valent metals, preferably magnetic particles of two- or three-valent iron oxides, optionally the adsorption is performed with mixture of these sorbents.

The sorbent is after the process of adsorption repeatedly washed with water or by a solution releasing the unattached proteins and unwanted parts of milk; the attached proteins are afterwards released by ionic strength. Lactoperoxidase and impurities such as immunoglobulins are eluted in the first step by ionic strength 0.01-0.3 mol.dm⁻³, then the residual lactoperoxidase is eluted by ionic strength 0.05-0.5 mol.dm⁻³ and afterwards lactoferrin by ionic strength 0.5-5 mol.dm⁻³; the ionic strength depends on acidity of the sorbent.

In optimal case, the isolated proteins are afterwards processed into the powder form by diaultrafiltration and by freeze-drying or spray-drying and the sorbent is regenerated by washing with water and chemical or temperature sterilization and repeatedly used in the separation process.

Finally, the advantage of using magnetic sorbent is a possibility to let the milk medium flowed to the further technological processes through the magnetic separator.

The process may be carried out in an apparatus for separation of whey proteins (the apparatus is not a part of the claimed invention). The apparatus contains a storage tank for milk medium with main mechanical stirrer and a drain. The main role of this system is the flowing of the media through a filtration mechanism with particles of the sorbent; the flowing keeps the particles in suspension. The filtration mechanism can be represented by filtration basket submersed in the medium. The filtration basket can be made by stainless wire mesh filters in the mesh size depending on the size of the sorbent in the range from 1 µm to 1 cm.

Preferably, the filtration mechanism is located inside the storage tank or the filtration mechanism can be also located outside in separation tank connected with storage tank via pipeline whereas feeding pump is located between storage and separation tank and flow pump is located behind the separation tank.

In optimum, the set-up of filtration mechanism is provided with second mechanical stirrer and a magnetic separator is located behind the separation tank.

In the comparison with traditional chromatographic-column separations, the presented technological solution provides better effectivity thanks to the simpler process of adsorption just by mechanical stirring of the sorbent in the whole volume of medium. The medium, e.g. fresh milk, milk protein concentrate, whey etc. does not have to pass under pressure through column or membrane as in typical chromatographic approaches. Hence, the presented solution prevents the separation from blocking and fouling of the separation unit (column, membrane) and the separation time is not dependent on the volume of the media. The presented technical solution also offers a separation of the proteins from fresh milk. Thanks to this possibility, Lf and LPO can be obtained in the best quality and quantity before pasteurization. The elution process is also easier. The volume of elution media is smaller in the case of releasing the proteins from the sorbent just by mechanical stirring in the elution media than by passing the medium through separation unit. The reduced volume also simplifies the post-preparative treatments of obtained proteins. In the comparison with the best current technological solution (chromatographic separation from milk protein concentrate), the presented basket-batch separation seems as non-invasive for implementation in to the dairy factories producing various types of milk products. The quality of Lf and LPO is comparable for both of these separation approaches. However, basket-batch separation using porous sorbents offers a possibility to separate from larger spectra of milk media. Thanks to this fact, this separation approach seems as a suitable alternative to current traditional separations but can also offer higher yields of isolated proteins per volume of processed milk with lower technological demands.

### Brief description of the drawings

The basics of technological solution and concrete solution are illustrated by attached figures:
Fig. 1: A schema of basic technological solution with filtration basket located in storage tank.
Fig. 2: A schema of alternative technological solution where the filtration basket is located in separation tank.
Fig. 3: a picture of magnetic cation-exchanger with -OSO₃H function used for separation from optical microscope, the size of the sorbent is from 50 to 80 µm.

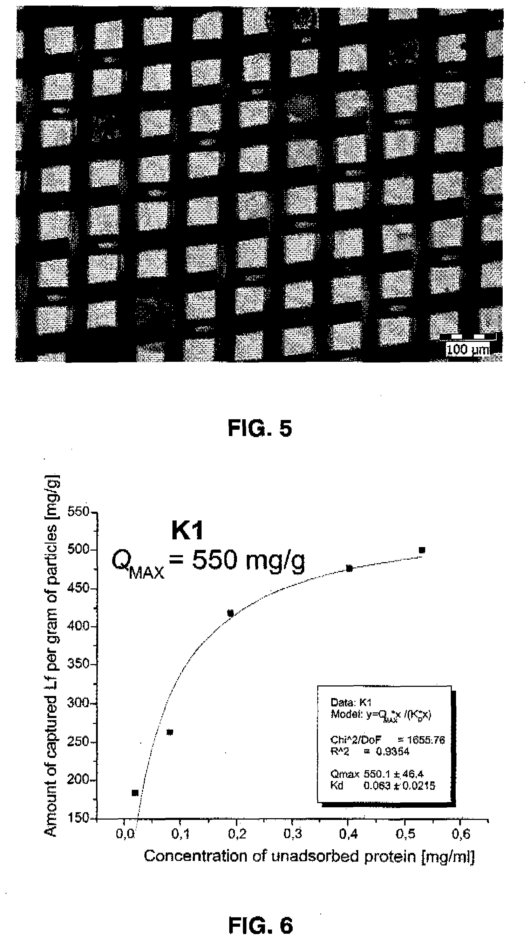

Fig. 4 and Fig. 5: the magnetic sorbent from picture 3 under and above stainless wire mesh filter with the mesh 50 µm.
Fig. 6: dependence of maximal capacity of Lf (i.e. mass of captured protein) on mass of sorbent with -SO₃H function.
Fig. 7: dependence of maximal capacity of Lf (i.e. mass of captured protein) on mass of sorbent with -COOH function.
Fig. 8: SDS-PAGE of isolated proteins obtained according the protocol in example 1 (I - protein markers in kDa scale, II - Lf standard, III - proteins in fresh milk: Immunoglobulins, Lf/LPO, bovine serum albumin, Immunoglobulins (large subunit), Immunoglobulins (small subunit), β-lactoglobulin, α-lactalbumin, IV - proteins in fresh milk after isolation of Lf and LPO, V - proteins eluted from sorbent by water, VI - 1. fraction according the protocol in example 1, VII - 2. fraction according the protocol in example 1, VIII - 3. fraction according the protocol in example 1.)

### Description of the invention

The approach for separation of Lf and LPO according technological solution is based on so called basket-batch separation of these proteins by typical chromatographic sorbents from fresh milk, skim milk, whey or another Lf containing media. This type of separation combines the possibility of batch separation as in case of magnetic separations, i.e. isolation via mechanical stirring of the sorbent in the whole volume of medium; subsequently, the separation uses the typical chromatographic sorbent which can be separated from the medium by filtering or potentially by external magnetic field. Hence, the sorbent can be repeatedly reused after elution of proteins.

The aim of this invention can be accomplished if the sorbent used for separation of Lf and LPO is typical sorbent for column separations, so the sorbent fulfills the following criteria:
a) The sorbent is based on polymer material which is functionalized by typical cation-exchange groups selected from -COO⁻, -OSO₃⁻ -PO₃²⁻, -OPO₃²⁻.
b) The polymer material of the sorbent is preferably hydrophilic through it is not soluble in water at standard conditions. The hydrophilic character is suitable for separation of proteins because it keeps the proteins in native form. Another important parameter is a possibility for further functionalization of the polymer for typical cation-exchange groups. Above all the material can be made from polysaccharides insoluble in water (e.g. agarose, cellulose, and modified dextran) or eventually from hydrophobic organic compounds typically used as stacionary phase in chromatographic columns for separation of biomacromolecules, e.g. sorbents prepared by polymerization of acrylamide, styrene, divinylbenzen, methacrylates etc.
c) The next point that should be accomplished for the aim of this invention is the mechanical stability of used sorbents as well as defined size distribution. These requirements are important for the ability of the sorbent to survive the process and also for possibility to remove the sorbent by filtration. In advanced, the sorbent defined by above mention points has the globular shape and the size in the range from tenths to hundredths of micrometers, e.g. with distribution from 50 to 80 µm or from 80 to 150 µm, for easy filterability by mechanical filters. Thanks to the spherical shape the sorbent has better mechanical stability.
d) Another important point of the spherical sorbent is the high porosity and high capacity for Lf and LPO.
e) The sorbent can be also a polymer particle with encapsulated magnetic material (e.g. magnetic nanoparticles of iron oxide or another material exhibiting reaction of external magnetic field. The magnetic part can afterwards act as a safety mechanism (to mechanical filtration mechanism) how to remove the sorbent from the medium.

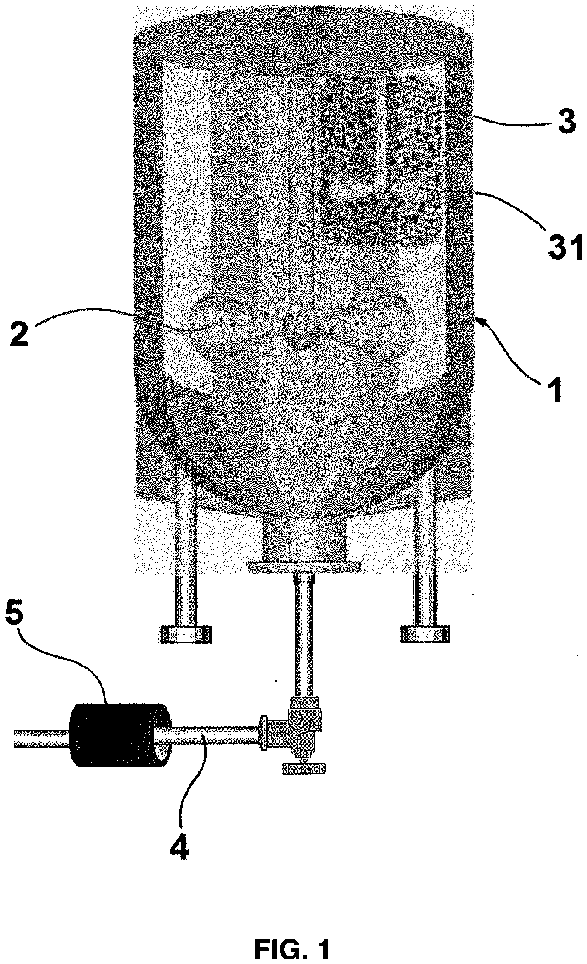

The basic set-up of presented way of separation is depicted in Fig. 1 (the apparatus is not a part of the claimed invention) and consists of storage tank (1) of milk medium, e.g. fresh milk, with the main mechanical stirrer (2); the filtration mechanism (3) is located in the storage tank. In advanced, the filtration mechanism is represented by filtration basket submersed in separation medium and made from stainless wire mesh filters in the mesh size depending on the size of the sorbent in the range from 1 µm to 1 cm. And safety magnetic separator (5) is incorporated after the drain (4) from storage tank (1) for 100 % separation of magnetic sorbent from the milk medium. Thanks to the circulation of medium in the storage tank (1), the medium also circulates inside the filtration mechanism (3) wherein a secondary mechanical stirrer (31) keeps the particles of sorbent in suspension and in intensive contact with the medium. The filtration mechanism (3) prevents from direct the contact of the sorbent with the whole medium; however it allows an interaction between both systems.

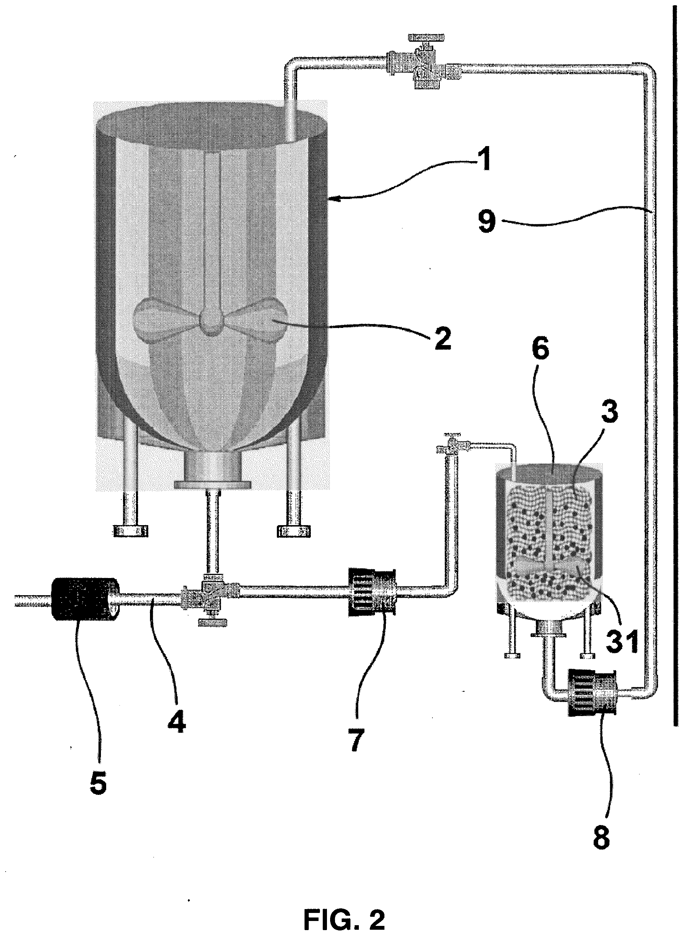

An alternative set-up is depicted in Fig. 2 (the apparatus is not a part of the claimed invention), the filtration mechanism (3), i.e. filtration basket, is incorporated in the separation tank (6) in a pipeline (9) located outside the storage tank (1), wherein the milk medium is pumped by feeding pump (7) which provides a circulation of the medium. The medium is afterwards pumped from the separation tank (6) back to the storage tank (1) by flow pump (8). The flow of medium through the separation mechanism (3) can be provided for the whole time of milk storage before its further processing.

The main aim of this technological solution, i.e. separation of Lf and LPO from milk media or other media containing Lf by basket-batch separation using mentioned sorbents with high capacity for Lf and LPO, can be accomplished by several simple operations.

The first step for separation of Lf and LPO is the adsorption of these proteins by sorbent, i.e. by cation-exchange interaction. The sorbent is mechanically stirred in whole volume of milk/whey even in large volumes in advance. The amount of isolated Lf/LPO depends on the capacity of used sorbent for the protein, on the ratio of magnetic sorbent to amount of medium and also on the temperature of medium and the time of interaction. The further important parameter that determines the purity of obtained proteins is value of pH of the medium, this value should be around 7 (e.g. 6.7 as the pH of fresh milk).

The sorption of proteins can be performed directly from fresh milk in storage tanks before its following processing as pasteurization, skimming etc. as is depicted on Fig. 1. The only one requirement is the filtration mechanism incorporated in/to the storage tank as filtration basket, i.e. a scaffold covered by stainless wire mesh filter. The sorption is guaranteed by mechanical motion of the sorbent however the sorbent is incorporated inside the filtration basket and cannot get into the next milk processes and is mechanically captured on the filter during milk draining. A magnetic separator can be also incorporated behind the drain in the case that magnetic separation has been used; it guarantees 100 % capture of the sorbent before the other processes. These magnetic sorbents are commercially available and are typically used food industry, e.g. chocolate production.

The basket-batch separation from fresh milk can be also performed at another set-up as is depicted on Fig. 2. This separation is based on the same principal as above mention separation, however the sorbent is located in another tank (approx. ten times smaller) with filtration basket with own mechanical stirrer. The fresh milk in this smaller tank is circulated by additional pumps; thereby the whole process is completely separated from the milk and its further manufacture processes are not affected. Magnetic separation can be afterwards placed just as a safety separation.

The following stage of separation is repeated washing with water for removal of unattached proteins and other unwanted part of milk. The following step is elution of adsorbed proteins. The elution can be performed similarly as the adsorption just by mechanical stirring of the sorbent in the elution medium. The elution can be done in smaller volume (more than ten times) in the comparison with the milk medium, in advance. Lf and LPO can be eluted from the sorbent at the same ionic strengths as in chromatographic separations. Unwanted immunoglobulins (isoelectric point higher than 7) can be eluted from the sorbent by low ionic strength, e.g. by ionic strength from 0.05 to 0.2 mol.dm⁻³ of NaCl at pH 7 depending on the acidity of the used sorbent. LPO is afterwards eluted by higher ionic strength, e.g. from 0.1 to 0.5 mol.dm⁻³, and Lf is eluted by solution of NaCl with ionic strength from 0.5 to 1 mol.dm⁻³. The obtained solutions of proteins are afterwards desalted (e.g. by diaultrafiltration) and dried into the form of powder (e.g. by freeze-drying or by spray-drying). After the separation, the sorbent is washed with water, sterilized, e.g. chemically or by temperature, and repeatedly used in the separation process.

The following examples show just a few types of Lf and LPO separation options using porous sorbents according the invention. Magnetic ion-exchanger based on cellulose with -OSO3H and -COOH function was used in the examples. However, other types of sorbent typically used in chromatographic separations (without magnetic part) are also disclosed . In the following examples, the fact that Lf and LPO can be separated from the fresh milk, the volume ratio of sorbent to milk medium, purity of separated fractions depending on the type and capacity of sorbent (strongly acidic or weakly acidic function) are illustrated.

The purity of Lf and LPO fractions was determined according the ratio of concrete protein concentration to total concentration of all proteins. Concentration of Lf was determined by bovine lactoferrine ELISA Kit, the concentration of LPO was determine by peroxidase (enzymatic) activity against ABTS, total concentration of all proteins was determined by Bradford analysis.

### Example 1

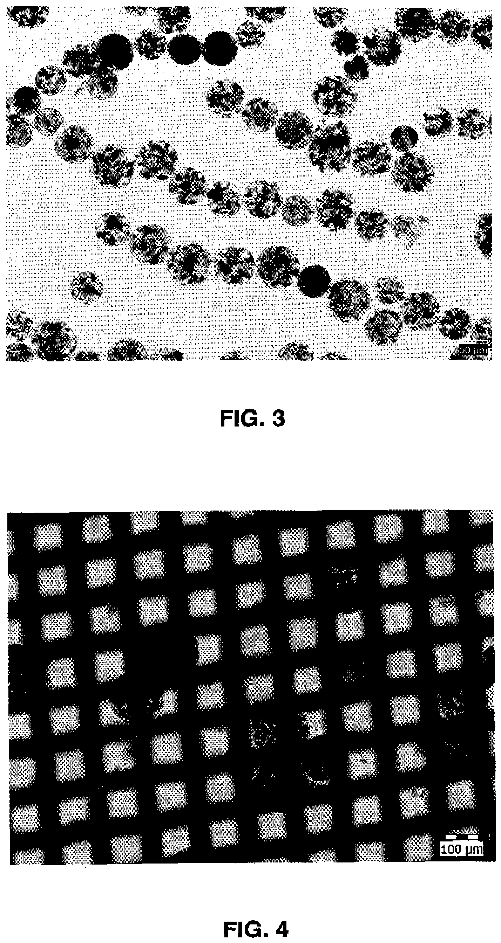

Commercially available magnetic cellulose sorbent with -OSO₃H functional group and with size distribution from 50 to 80 µm (0.8 mL in concentrated form) was three times washed by water and redispersed in 50 mL of fresh milk (without pasteurization). The pH value of resulted suspension was 6.7. The mixture was stirred at room temperature for the next 2 hours. The magnetic sorbent was separated by hand magnet and three times washed with water. Afterwards, the adsorbed proteins were eluted by mechanical stirring (10 minutes) by three step gradient of ionic strength (5 mL of NaCl solution, pH 7). In the first step, 0.1 M solution of NaCl was used for elution of LPO and basic immunoglobulins; the purity of obtained LPO was 60 % and the yield was 1 mg. In the second elution step, 0.6 mg of LPO with purity 85 % was obtained. In the last elution step, 4 mg of Lf with purity ≥95 % was eluted. SDS-PAGE of obtained proteins is depicted on the Fig. 3. The magnetic sorbent was afterwards washed with water a repeatedly used at same conditions as above with similar yields. The milk after the separation was tested on milk fatness and acidity. The milk before the separation and after the separation had the same values of fatness and acidity.

### Example 2

Adsorption and elution of Lf and LPO was performed at the same conditions as in example 1. However, the magnetic sorbent was stirred in fresh milk at the temperature range from 2 to 8 °C. The time of mechanic stirring was increased to 4 hours. The first fraction of proteins was LPO (0.8 mg, 65 % purity), second fraction obtained 0.45 mg of LPO with 80 % purity and the last one 4.2 mg of Lf with ≥95 % purity.

### Example 3

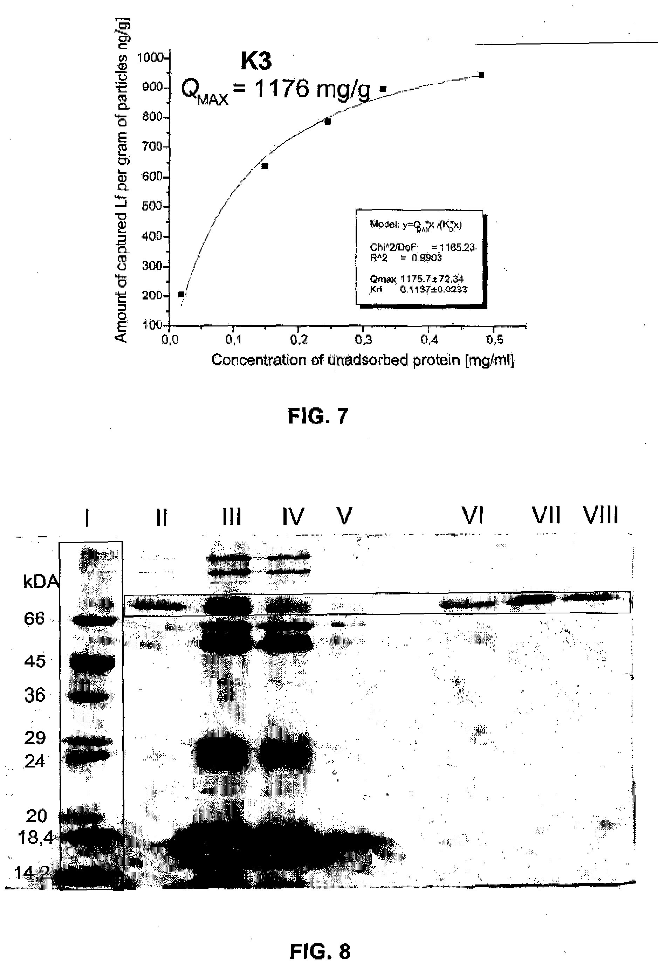

Adsorption and elution of Lf and LPO was performed at the same conditions as in example 1. Just the magnetic sorbent was changed; magnetic sorbent with weakly acidic function (-COOH) was used instead the strongly acidic function (-OSO₃H). The magnetic ion-exchanger exhibited higher maximal capacity for Lf (see Fig. 7). The magnetic sorbent (2 mL) was used for separation from 200 mL of fresh milk. The adsorption and elusion was performed according the protocol in example 1. This strategy provided 3.2 mg of LPO (55 % purity) in first fraction, 2.9 mg of LPO (70 %) in second fraction and 12 mg of Lf (≥95 % purity) in third fraction.

### Example 4

Adsorption and elution of Lf and LPO from 200 mL of fresh milk was performed at the same conditions as in example 1. Magnetic ion-exchanger with medium acidic group (-O-PO₃H₂) was used as the sorbent. LPO (5 mg, 45 % purity) was eluted in the first fraction (0.05 M ionic strength), in the second fraction (0.2 M) 2.8 mg of LPO with 80 % purity was eluted. In the last step, 15.8 mg Lf with purity ≥95 % was obtained.

The presented examples clearly show that the magnetic separation provides good yields and can be used not only in whey with minimal concentration of Lf but also in the fresh milk. Magnetic sorbent with strongly acidic -OSO₃H group seems as the most suitable for separation of Lf and LPO by mechanical stirring in the milk (by batch separation). This sorbent can reach sufficient yields and also good purity of isolated proteins. The purities are similar as in the industrial chromatographic separations. Another important parameter is that the main parameters of milk stay unchanged during the separation, i.e. milk acidity and fatness, and the purity and yields of isolated proteins are reproducible in the repeated cycles.

### Industrial applicability

The presented basket-batch separation of Lf and LPO can find its applicability mainly in dairy industry dealing not only with production of daily products, e.g. butter, cheese, yogurts etc., but also in dairy industry dealing with isolation of bioactive substances from milk, e.g. colostrum. The purified proteins can be afterwards used for production of food supplements, cosmetics or other bioactive substances of drugs in pharmaceutical industry.

## Claims

1. A method for separation of whey proteins lactoferrin and lactoperoxidase, from milk media, preferably from fresh milk or whey, by sorbents, wherein the milk medium is in contact with the sorbent by mechanical stirring; the sorbent is porous and magnetically filterable, made from polymer material functionalized by cation-exchange groups selected from -COO⁻, -OSO₃⁻, -PO₃²⁻, and -OPO₃²⁻, and the sorbent contains a magnetic part composed of inorganic metal compounds or zero-valent metals, said sorbent is kept in suspension in a separate filtration space with filtration mechanism, preferably in filtration basket, and adsorbs whey proteins, whereupon the filtration mechanism with adsorbed proteins is taken out and after wash-out of unattached proteins and other unwanted parts of milk medium the attached whey proteins are eluted, wherein after the removal of unattached proteins by deionized water the adsorbed whey proteins are released from the sorbent by ionic strength,
in the case that the milk medium contains lactoperoxidase and lactoferrin, lactoperoxidase and impurities such as immunoglobulins are eluted in the first step by ionic strength 0.01-0.3 mol.dm⁻³, then the residual lactoperoxidase is eluted by ionic strength 0.05-0.5 mol.dm⁻³ and afterwards lactoferrin by ionic strength 0.5-5 mol.dm⁻³;
and afterwards processed into the powder form.

2. The method for separation of whey proteins according to claim 1, wherein the adsorption is performed for time period 0.1-24 hours, preferably 2-6 hours, at temperature range 1-85 °C.

3. The method for separation of whey proteins according to claims 1 and 2 wherein the adsorption is performed with sorbent wherein said magnetic part being magnetic particles of two- or three-valent iron oxides.

4. The method for separation of whey proteins according to claims 1-3, wherein the isolated proteins are processed into the powder form by diaultrafiltration and by freeze-drying or spray-drying.

5. The method for separation of whey proteins according to claims 1-3, wherein the sorbent after the separation is regenerated by washing with water and chemical or temperature sterilization and repeatedly used for the separation.

## Patentansprüche

1. Verfahren zur Trennung von Molkeproteinen Lactoferrin und Lactoperoxidase aus Milchmedien, vorzugsweise aus Frischmilch oder Molke, durch Sorptionsmittel, wobei das Milchmedium durch mechanisches Rühren mit dem Sorptionsmittel in Kontakt steht; Das Sorptionsmittel ist porös und magnetisch filtrierbar, hergestellt aus Polymermaterial, das durch Kationenaustauschgruppen, ausgewählt aus -COO⁻, -OSO₃⁻, -PO₃²⁻ und -OPO₃²⁻, funktionalisiert ist und das Sorptionsmittel einen magnetischen Teil enthält, der aus anorganischen Metallverbindungen oder Nullvalenten Metallen, wobei das Sorptionsmittel in einem separaten Filtrationsraum mit Filtrationsmechanismus, vorzugsweise im Filtrationskorb, suspendiert ist und Molkeproteine adsorbiert, woraufhin der Filtrationsmechanismus mit adsorbierten Proteinen herausgenommen wird und nach dem Ausspülen von ungebundenen Proteinen und anderen Unerwünschte Teile des Milchmediums werden die angebrachten Molkenproteine eluiert, wobei nach der Entfernung von ungebundenen Proteinen durch deionisiertes Wasser die adsorbierten Molkeproteine aus dem Sorptionsmittel durch Ionenstärke freigesetzt werden,
In dem Fall, dass das Milchmedium Lactoperoxidase und Lactoferrin enthält, werden Lactoperoxidase und Verunreinigungen wie Immunglobuline im ersten Schritt durch Ionenstärke 0,01-0,3 mol.dm⁻³ eluiert, dann wird die restliche Lactoperoxidase mit einer Ionenstärke von 0,05-0,5 mol.dm⁻³ eluiert und danach Lactoferrin durch Ionenstärke 0,5-5 mol.dm⁻³ eluiert wird;
und danach in die Pulverform verarbeitet.

2. Verfahren zur Trennung von Molkeproteinen nach Anspruch 1, **dadurch gekennzeichnet, daß** die Adsorption für den Zeitraum 0,1-24 Stunden, vorzugsweise 2-6 Stunden, bei einem Temperaturbereich von 1-85 °C durchgeführt wird.

3. Verfahren zur Trennung von Molkeproteinen nach Anspruch 1 und 2, wobei die Adsorption mit Sorptionsmittel durchgeführt wird, wobei der magnetische Teil magnetische Partikel von zwei- oder dreivalenten Eisenoxiden ist.

4. Verfahren zur Trennung von Molkeproteinen nach den Ansprüchen 1-3, wobei die isolierten Proteine durch Diaultrafiltration und durch Gefriertrocknung oder Sprühtrocknung in die Pulverform verarbeitet werden.

5. Verfahren zur Trennung von Molkeproteinen nach den Ansprüchen 1-3, wobei das Sorptionsmittel nach der Trennung durch Waschen mit Wasser und chemischer oder Temperatursterilisation regeneriert und wiederholt für die Trennung verwendet wird.

## Revendications

1. Procédé de séparation des protéines de lactosérum, la lactoferrine et la lactoperoxydase, à partir des milieux du lait, de préférence du lait frais ou du lactosérum, par des sorbants, dans lequel le milieu laitier est en contact avec le sorbant par agitation mécanique; le sorbant est poreux et magnétiquement filtrable, fabriqué à partir de matériau polymère fonctionnalisé par des groupes échangeurs de cations sélectionnés parmi -COO⁻, OSO₃⁻, -PO₃²⁻ et -OPO₃²⁻, et le sorbant contient une partie magnétique composée de composés métalliques inorganiques ou métaux de valence zéro, ledit sorbant est maintenu en suspension dans un espace de filtration séparé avec un mécanisme de filtration, de préférence dans un panier de filtration, et adsorbe les protéines de lactosérum, après quoi le mécanisme de filtration avec des protéines adsorbées est retiré et après lavage des protéines non liées et autres parties indésirables du milieu du lait, les protéines de lactosérum attachées sont éluées, après quoi, après l'élimination des protéines non attachées par de l'eau désionisée, les protéines de lactosérum adsorbées sont libérées du sorbant par la force ionique,
dans le cas où le milieu laitier contient la lactoperoxydase et la lactoferrine, la lactoperoxydase et les impuretés telles que les immunoglobulines sont éluées dans la première étape par une force ionique de 0,01 à 0,3 mol.dm⁻³, puis la lactoperoxydase résiduelle est éluée par une force ionique de 0,05 à 0,5 mol.dm⁻³ et ensuite la lactoferrine est éluée par une force ionique 0,5-5 mol.dm⁻³;
et ensuite transformées en forme de poudre.

2. Procédé de séparation de protéines de lactosérum selon la revendication 1, dans lequel l'adsorption est effectuée pendant une période de temps de 0,1 à 24 heures, de préférence de 2 à 6 heures, à une température de 1 à 85 °C.

3. Procédé de séparation de protéines de lactosérum selon les revendications 1 et 2, dans lequel l'adsorption est effectuée avec du sorbant, ladite partie magnétique étant des particules magnétiques d'oxydes de fer de valence deux ou trois.

4. Procédé de séparation des protéines de lactosérum selon les revendications 1 à 3, dans lequel les protéines isolées sont transformées en forme de poudre par diaultrafiltration et par lyophilisation ou séchage par pulvérisation.

5. Procédé de séparation des protéines de lactosérum selon les revendications 1 à 3, dans lequel le sorbant après la séparation est régénéré par lavage avec de l'eau et une stérilisation chimique ou à température élevée et utilisé à plusieurs reprises pour la séparation.
